# EUROPEAN PATENT APPLICATION

(11) **EP 1 887 080 A1**
(43) Date of publication of application: **13.02.2008**
(21) Application number: 06743497.7
(22) Date of filing: 12.05.2006
(51) Int. Cl.: C12N 5/10, C12N 15/18, A61F 2/10, A61L 27/60

(54) **ENDOTHELIZED ARTIFICIAL MATRIX COMPRISING A FIBRIN GEL, WHICH IS A SUPERPRODUCER OF PROANGIOGENIC FACTORS**

(30) Priority: 16.05.2005 ES 200501182
(71) Applicant: Fundacion para la Investigacion Biomedica Del Hospital Gregorio Marañon, 28007 Madrid (ES)
(72) Inventor: LASSO VÁZQUEZ, José Maria, E-28006 Madrid (ES); NAVA PEREZ, Paola, E-28007 Madrid (ES)
(74) Representative: Elzaburu Marquez, Alberto
(86) International application number: PCT/ES2006/070059
(87) International publication number: WO 2006/123004

(57) **Abstract**

Endothelized artificial matrix comprising a fibrin gel which is a superproducer of proangiogenic factors. The matrix comprises a fibrin gel containing embedded endothelial cells that have been transfected *in vitro* with at least one adenoviral vector containing the sequence encoding at least one proangiogenic factor inserted in such a way that it can be over-expressed in these endothelial cells. The insertion of the aforementioned matrix between a flap and its recipient bed in a transplant process improves the survival rates of said flap, as the endothelized matrix is able to induce angiogenesis both in the flap and the recipient bed, thus improving the vascularisation of the transplanted area.

## Description

### TECHNICAL FIELD

The present invention applies to the field of artificial matrices prepared from polymeric substances present in nature, where cells are seeded and made grow for their subsequent use in plastic and reconstructive surgery.

### BACKGROUND OF THE INVENTION

In the last few years, the development of microsurgical techniques, complemented with improved knowledge of anatomy, has been one of the great advances that have benefited Plastic and Reconstructive Surgery. Despite this, when reconstructions with flaps are made, there is a variable risk of necrosis of the same, in many cases due to vascular disturbances. The flaps are own tissues (consisting of skin, muscles, bones or a combination of the same) which can be placed in anatomical areas where, due to oncological or traumatic processes, among others, a defect has been produced that requires reconstruction. Flaps have to be used when the losses of the cutaneous substance or subcutaneous tissue are not suturable or cannot heal spontaneously. The purpose of the flap is to close a loss of substance or reconstruct an amputated structure. A skin flap is a piece of skin and subcutaneous cellular tissue that maintains autonomous vascularisation through the pedicle, with which it remains in contact with the deep structures. The flap pedicle is the cutaneous bridge that directly irrigates the same; sometimes it is reduced and may be represented by an artery and one or two veins. The flap is called local when the tissue that it is made from is obtained in an area near the defect that is to be repaired, and called a distant flap when the tissues are obtained from areas remote from the defect. In this last case, the flap has one artery and one vein which have to be anastomosed to another vein and artery, respectively, from the anatomical area where it is going to be located.

Thrombotic events, in both the venous and the arterial zone, and even in the micro-vessels, are the biggest problems that have to be confronted when performing a reconstruction with flaps, their appearance rate being higher in distant flaps, as they depend on microsuturing vessels of 2 mm to 5 mm in diameter. In these cases the pedicle has to be moved from its site of origin, and has to be resutured to local vessels near the area that requires reconstruction, which increases the morbidity of the process. For this reason, different methods have been sought to decrease the rate of thrombosis. There are clinical and research studies with drugs that reduce the thrombogenic potential, such as platelet antiaggregants, anticoagulants or thrombolytic agents.

Angiogenesis is the formation of new capillaries from already existing ones. It is a complex process, which may be activated in response to tissue damage. The factors involved in its stimulation are called proangiogenic factors; they play a key role in the wound healing process, decisively orchestrating the dermal neovascularisation phase. Of those, one part appears to be growth factors that are capable of stimulating the *in vivo* proliferation and migration of the cells that take part in the formation and stabilisation of blood capillaries. In the field of clinical practice in reconstructive surgery, growth factors also have an important role for stimulating healing in deficiency or complicated states, as happens in diabetic, oncology, and malnourished patients or those who have suffered severe traumas, in those where stress leads to a lack of all the factors that influence healing and, also, it usually increases the thrombosis risk due to their poor general state, as well as to medications, prolonged bed confinement... In diabetic patients in particular, neuropathy is produced, which changes the functioning of the blood vessels, or microangiopathy, which obstructs the blood capillaries, leading to a deficit in tissue perfusion which then leads to destruction of the tissue that these vessels nourish, thus the need for providing local factors that accelerate the incorporation of, for example, a flap to the bed where it is going to be transplanted should help to increase the vascular connections between the bed and the flap and thus increase the survival rate. The role growth factors play in tissue regeneration is also important, such as, for example, when working with prefabricated flaps.

Among the growth factors that appear to be involved in the regulation of angiogenesis, fibroblast growth factors (FGF), platelet derived growth factors (PDGF), transforming growth factor alpha (TGF-alpha) and hepatocyte growth factor (HGF), can be mentioned. Also, it has been suggested that a specific endothelial cell growth factor, vascular endothelial growth factor (VEGF) is responsible for the stimulation of growth and differentiation of endothelial cells, and certain functions of differentiated cells.

The existence of FGF (fibroblast growth factor) in the brain and in the pituitary was established by Gospodarowicz in 1974. Today, it is known that FGFs represent a group of similar proteins that act as powerful mitogens for some mesodermal and ectodermal cells.

Fibroblasts are the most common cells in connective tissue and are adhesion cells that play an important role in aiding the healing process. The stabilisation of collagen in healing is promoted by the introduction of FGF in the wound bed, which appears to help in the viability of the blood vessels and promote fibroblast activity.

The angiogenic effect of FGF has also been shown in other studies. Lu and coworkers ((Lu WW et al., Br J Plast Surg, 53: 225-229, 2000) observed that there was less ischaemia and less changes in the distribution of collagen in wounds treated with FGF, which led to a higher ability to support strain and a higher elasticity of the tissues.

The structure and functions of acidic and basic FGF are known. Basic FGF is localized in the brain, hypophysis, retina, kidneys, corpus luteum, placenta, prostate, adrenal cells and macrophages. Acidic FGF is localized in the brain and retina. Both stimulate endothelial cell migration and proliferation. There are studies that demonstrate the angiogenic ability and improvement in viability of flaps treated with FGF, whether the aforementioned is injected subcutaneously (Im MJ et al., Ann Plast Surg, 28: 242-245, 1992) or if it is repeatedly applied using slow release pellets (Less VC et al., Br J Plast Surg 47: 349-359, 1994). In melanomas it is capable of producing angiogenesis along with other growth factors (Rofstad EK et al., Cancer Res, 60: 4719-4724, 2000) and it seems that it could promote survival and branching of myocardial arteries (Carmeliet P, Cir Res, 87: 176.178, 2000).

VEGF, for its part, was initially described as a protein secreted by tumour cells, which increased the permeability of the local vessels to circulating macromolecules. It is produced by different cells in the body, among them, endothelial cells, on which it specifically acts. The direct actions of VEGF are numerous and include, among others, an increase in endothelial cell permeability. Compared to histamine, VEGF is 50,000 times more powerful as far as vascular permeability is concerned. The administration of topical VEGF produces fenestrations in the endothelium of the micro-vessels and capillaries (Roberts WG et al., J. Cell Sci, 108: 2369-2379, 1995).

During the healing process, the production of VEGF form keratinocytes is increased. This also happens in the mononuclear cells in the region where healing is taking place (Tabú PJ et al, Plas Reconst Surg, 105: 1034-1041, 2000). Under physiological conditions, its production is induced by the decrease in tissue oxygen tension. The half life of VEGF under normal conditions is from 30 to 45 minutes, but under hypoxia conditions its production is extended to 6-8 hours, depending on its level of production by the tissue which is subjected to ischaemia, and the extent of tissue affected. Its production can also be increased in several diseases (Akagi K et al., Br J Can, 83: 887-891, 2000; Philipp W et al., Invest Ophtalmol Vis Sci, 41: 2514-2522, 2000).

In ischaemic areas, the endothelial cells are capable, in response to VEGF (initially liberated by inflammatory cells), of synthesising more VEGF, as well as increasing the density of the receptors for this factor in their membranes. For this reason, in an emergency situation such as ischaemia, the endothelial cells behave at the same time as producers and as targets of VEGF, thus generating a chain and amplified reaction to the factor.

Several experiments have been carried out with VEGF in plastic surgery, with the aim of improving tissue perfusion. Padubiri et al (Padubiri A et al., Ann Plast Surg, 37: 604-611, 1996) injected VEGF (as recombinant protein) into the pedicle of an abdominal flap and subsequently produced an ischaemia in the same. After 7 days, the subjects treated with VEGF had a flap survival higher than those not treated. Similarly, Banbury and coworkers (Banbury J et al., Plast Reconst Surg, 106: 1541-1546, 2000) demonstrated that it was possible to improve the perfusion of muscular flaps (cremaster muscle, in rats) subjected to ischaemia when these same rats received treatment with a VEGF perfusion in the sub-critical phase.

Studies have also been carried out to try to find the best administration route for growth factors. Kryger's group (Kryger Z et al., Br J Plast Surg, 53: 234-239, 2000) designed a rat study, with the objective of comparing different administration routes for VEGF in flaps. They designed six treatment groups, which were distinguished by being treated as follows: a single systemic dose of VEGF, multiple doses systemically, subcutaneously, subfascially and topically and a final control group, treated with normal saline. The best results were obtained in the group treated with multiple systemic doses of VEGF, over 72 hours. The worst result was obtained with the group treated topically with VEGF. VEGF has also been used in prefabricated flaps. This factor appeared to accelerate the maturing of these flaps when administered to rats using polyvinyl alcohol gel (Li QF et al., J Reconst Microsurg, 16: 45-50, 2000).

Although the results are promising, the use of growth factors such as recombinant proteins has a clear limitation, which is its short half life *in vivo.* Although growth factors are only needed temporarily, until the resolution of the defect, it is fundamental to obtain a therapeutic effect that the bioavailability of the factor is guaranteed during this temporary period. One of the strategies used to overcome this obstacle has been to resort to repeated doses of the factor for a brief lapse (Kryger Z et al., Br J Plast Surg, 53: 234-239, 2000). A probably more efficient alternative would be to apply the growth factor not as a protein, but as a gene that is continuously expressed until the process is complete.

For this reason, the introduction of gene therapy techniques in the field of reconstructive surgery and in wound healing is of great use. Although the techniques for applying gene therapy are diverse and advancing rapidly, they mainly use viral vectors and liposome or plasmid complexes (Patterson C et al., Circulation, 102: 940-942, 2000). Adenoviruses are among the viruses being studied for use in gene therapy. They form part of a group of similar viruses, of which 47 serotypes are known. Serotypes 2 and 5 are the ones most used in gene therapy. They are double chain DNA viruses, with an icosahedral capsid. In their cycle, the viral genome resides in the nucleus, as an episomal element. They are capable of infecting a wide variety of cells.

According to Oligino (Oligino TJ et al., Clin Orth, 379S: S17-30, 2000), the efficiency of the infection by adenoviruses is high, compared to the lentiniviruses or adeno-associated viruses, although it is lower than that of the herpes viruses. Adenoviruses are not integrated in the genome of transduced cells and the duration of the transgene expression is transient, although very high. Large scale production is relatively easy. Adenovirus carriers of the VEGF gene have been used to treat patients with ischaemia of the limbs (Laitinen M et al., Hum Gene Ther, 9: 1481-86, 1998; Isner JM et al., Lancet, 348: 370-374, 1996), with a good tolerance by the patients and with no local inflammation or adverse effects. The application route was intra-arterial, although the presence of anatomical barriers, such as the lamina interna or arteriosclerosis usually reduces its efficacy. The production of growth factors with this technique generally reaches a peak at one week after treatment and the effect usually disappears at four weeks (Ylä-Hettuala S, Curr Opin Lipidol, 8: 72-76, 1997).

Another strategy for using adenoviruses as vectors to provide genetic material to angiogenesis promoter cells are described in the document WO 02/36131, in which it promotes the transfection by two adenoviral vectors, each one of them containing a different form of VEGF (VEGF-B₁₆₇ and VEGF-A), by injecting it in mouse ears. As with the use of adenovirus VEGF carriers mentioned in the previous paragraph, the transfection is produced *in vivo,* therefore the angiogenesis promoter action, although it is supposed to occur in endothelial cells, is really non-specific; injections of the adenoviruses are carried out in the blood vessels of the area to treat; therefore they were able to be systemically dispersed, with possible adverse effects; moreover, although there is increased VEGF synthesis in the first hours (24-48 hours), the effect is not maintained, an effect for which repeated inoculations of the adenovirus are required over several days, with the subsequent discomfort to the hypothetical patient.

It would be worthwhile having an administration method available for angiogenic factors encoded by viral carriers where the transfection is produced in *vitro,* thus permitting this transfection to be specific for endothelial cells and avoiding injecting the viruses into the blood vessels. Also, it would be advantageous if that method would enable the release of VEGF (or another proangiogenic factor) to be maintained over days with a single inoculation of viral vectors, making it possible for the proangiogenic factor to be available in sufficient quantities throughout the whole period of time that would be required to promote angiogenesis, but without requiring the inconvenience of repeated doses of that vector. For this reason, the matrices of fibrin gels where the cells are made to grow are a very suitable vehicle. The fibrin provides a good base for the growth of both dermal and epidermal cells, reason why this protein has often been used as a support for culturing keratinocytes (Ronfard et al., Burns 17:181-184, 1991). As the fibrin does not interfere with the subsequent development of the correct dermal/epidermal binding between a wound bed and the cultured keratinocytes, it has been widely used as a transport system for the aforementioned keratinocytes with the objective of repairing cutaneous lesions (Pellegrini et al., Transplantation 68: 868-879, 1999; Kaiser & Stark, Burns 20: 23-29, 1994).

Fibrin has also been used as a dermal base destined for producing large surfaces of cultured skin (Meana et al., Burns 24: 621-630, 1998). The seeded fibroblasts are able to grow inside the fibrin gels. At the same time, these fibroblasts behave as inducers of keratinocyte growth, so that, by seeding fibroblasts and a very limited number of cultured keratinocytes over a fibrin gel, stratified confluent epithelia, very similar to normal human epithelia, are obtained in a few days (Spanish Patent ES2132027). As described in the European patent application EP 1375647, the results can be improved by using human plasma as a fundamental base for the extra-cellular matrix, which includes platelets in its composition, resuspending dermal fibroblasts in the same to obtain an artificial dermis after coagulation of the plasma, on which dermis keratinocytes are seeded which adhere, migrate and grow in such a way that, in a few days, a tissue consisting of two parts is obtained, an upper one, consisting of stratified epithelial cells, and a lower one, consisting of an extra-cellular matrix densely populated with fibroblasts.

With the purpose of using skin analogues similar to those described previously in transplant processes where it is attempted to replace damage skin with the aforementioned analogues, it has been proven with different strategies where there is an attempt to increase the presence of substances that take part in angiogenesis with the aim of improving the chances of success of the artificial skin transplant. Thus, for example, the introduction of microspheres coated with fibroblast growth factor (FGF) into artificial dermis has been described (Kawai K et al., Biomaterials 21: 489-499, 2000), or inducing the production of recombinant proteins involved in angiogenesis by means of the genetic modification of keratinocytes with retroviral vectors carrying the genes of, for example, leptin hormone (WO 03/002154), VEGF (Supp et al., J Invest Dermatol 114: 5-13, 2000; Del Río M et al., Gene Therapy 6: 1734-1741, 1999) or FGF (Erdag G et al., Molecular Therapy, 10: 76-85, 2004) or by the genetic modification of fibroblasts with vectors carrying genes that express TGF (WO 02/030443) or other angiogenic factors (WO 03/095630). However, there are no cases described where the modification of cells included in a fibrin matrix or grown on a fibrin matrix has been produced with adenoviruses or cases where the cells that are genetically modified and made to grow on a fibrin matrix are endothelial cells. The nearest to this latter case would be the approach described in US5674722, where it is described the transfection of endothelial cells so that they might synthesise some non-specific protein, using as vectors, not adenoviruses, but retroviruses; also, the purpose described for the transfected cells, is not to culture them on a fibrin matrix, but to coat a synthetic material that is shaped like a blood vessel. Except in this last case, in all the rest of the works mentioned the final purpose of the generated matrix with cells is to obtain a skin analogue which could be transplanted as such in patients with lesions, without describing in any case its insertion jointly with a flap obtained directly from the anatomy of the patient to treat.

The present invention, however, proposes a different approach: the development of vascularised bridges made of fibrin gel matrices invaded by endothelial cells, superproducers of proangiogenic factors due to having been transfected *in vitro* with adenoviral vectors that contain genes that code them, with the purpose that the fibrin matrix that contains the endothelial cells acts as a bridge that could be inserted between flaps of any composition (skin, muscles, bones or a combination of the same) and the anatomical part requiring reconstruction, to improve the success of the implant process. By using the aforementioned endothelized matrix as a vascularised bridge, the incorporation of the skin, muscle or bone flap to the recipient bed is accelerated, because angiogenesis increases both in the transplanted tissues and in the recipient bed itself, the latter being an advantage that is particularly important in diabetic, malnourished subjects, those who have suffered severe traumas or who have been treated with radiotherapy (for example, mastectomised women, with radiotherapy treatment, who are going to receive a reconstruction with a musculo-cutaneous free flap); they are subjects among them failure rate in flaps is usually greater, because the tissues are poorly irrigated, as explained previously, a medium like the endothelized fibrin matrix of the invention, which facilitates the formation of vascular bridges between a flap and the recipient bed, being of special importance in these cases. On placing the fibrin gel matrix with the endothelial cells between the flap and the irradiated area, the angiogenesis produced by the gel should affect both in the same way and vascular bridges would be established between the two tissues in the first few hours after surgery. Besides, the angiogenic effect is a local and more specific effect than that obtained by injecting adenoviruses carrying genes encoding growth factors into the blood vessels, avoiding the risk of systemic dispersal, due to the transfection of the endothelial cells with the adenovirus vectors having been performed *in vitro,* before obtaining and implanting the vascularised bridge. The releasing of the growth factor, on the other hand, continues for days, and repeat doses are not necessary, which is more convenient for the patient. Also, unlike what might happen if the used vectors derive from a retrovirus, the use of adenoviral vectors eliminates the risk that, along with the inactivated retroviruses generated to act as carriers of the coding sequences of interest, non-inactive retroviral genetic material is packed and, therefore, with the potential of being wholly integrated in the host cell, blocking genes of interest or genes that could give rise to an oncogenic process after being blocked.

### DESCRIPTION OF THE INVENTION

The invention refers to an endothelized matrix envisaged to be used as a vascularised bridge, composed of a fibrin gel, which supports in its interior endothelial cells capable of synthesising VEGF and/or FGF under conditions in which their synthesis would not be induced under normal conditions, due to having been transfected *in vitro* with adenoviral vectors carrying genes that encode the aforementioned proteins. Due to those transfected genes, these endothelial cells will express amounts of VEGF that will be higher than those produced during the normal angiogenic process that would take place in an individual recipient of a flap in a normal transplant process, reason why they have been labelled as "superproducers" of angiogenic factors in the present application.

The object of its development is to recreate, in the laboratory, in a highly efficient way, a situation similar to that which occurs *in vivo* in an ischaemic area, as the aforementioned matrix invaded by endothelial cells would mimic the first stages of migration and proliferation that take place *in vivo* in an ischaemic area. Once obtained, the final purpose of the endothelized matrix is its insertion as an intermediate element between a flap used in the reconstruction process and the recipient bed that receives it, such that, after the transplant, the matrix inserted like a vascular bridge acts as a strong inducer of angiogenesis on the recipient individual. To increase the performance of the system, the endothelial cells, before being seeded in the matrix, are converted into superproducers of proangiogenic factors (VEGF and/or FGF) using a gene transference protocol with adenoviral vectors that carry its coding sequence and control elements that enables them to be expressed in endothelial cells. The matrix used, a fibrin gel, acts as an optimal support for cell proliferation and migration as well as for the production of the factors.

This system, which combines the introduction of endothelial cells into the matrix with the sustained *in situ* production (but for a limited time) of proangiogenic factors, represents a clear advantage over the topical or systemic administration of growth factors as recombinant proteins or by injecting adenoviral vectors that contain genes encoding the aforementioned recombinant proteins with the aim of obtaining an *in vivo* transfection process.

Another object of the present invention is a method for the production of the aforementioned endothelized fibrin matrix, superproducer of at least one proangiogenic factor due to its endothelial cells having been partly or completely transfected *in vitro* with one or more adenoviral vectors which have at least one gene corresponding to a proangiogenic factor in their sequence, comprising the steps of:
a) obtaining individualised endothelial cells after having been isolated from a mammal and cultured *in vitro;*
b) transfecting *in vitro* a part or all the mentioned endothelial cells with one or more different adenoviral vectors which comprise in their sequence at least one gene corresponding to a proangiogenic factor inserted in such a way that the gene is able to be expressed in endothelial cells;
c) mixing the medium that contains the endothelial cells transfected in the previous step with a solution that contains fibrinogen and stimulating the gelling of the fibrinogen to form fibrin;
d) allowing the mixture from the previous step to stand in a suitable receptacle so that the formation of the fibrin gel matrix is produced in which the endothelial cells transfected with adenoviral vectors have resulted embedded.

Similarly, it is an object of the invention to use the endothelized matrix which is a superproducer of proangiogenic factors as a vascularised bridge to be inserted between a flap and a recipient bed of the same, to improve the survival of the said flap.

In a preferred embodiment of the invention, the endothelized fibrin matrix which is a superproducer of proangiogenic factors will be designed with the aim that the recipient individual for whom it is envisaged would be human.

In a embodiment of the invention, the individual from whom the endothelial cells as well as the fibrinogen from which the fibrin derives is the same as that envisaged as the recipient individual of the endothelized matrix, the matrix being completely autologous.

In another embodiment of the invention, the matrix is not autologous, individuals different from the one envisaged as the matrix recipient being possible as donors of endothelial cells and/or fibrinogen. The donor and recipient individuals could even belong to different species.

### SHORT DESCRIPTION OF THE FIGURES

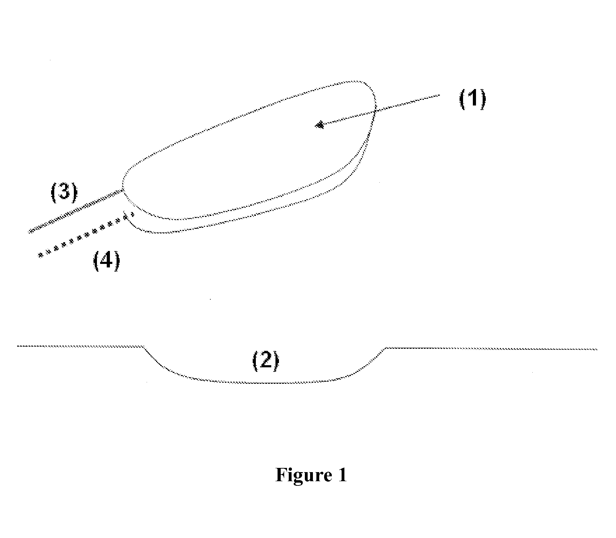

Figure 1 shows a schematic representation of a dissected flap. (1): flap; (2): avascular bed; (3): artery; (4): vein.

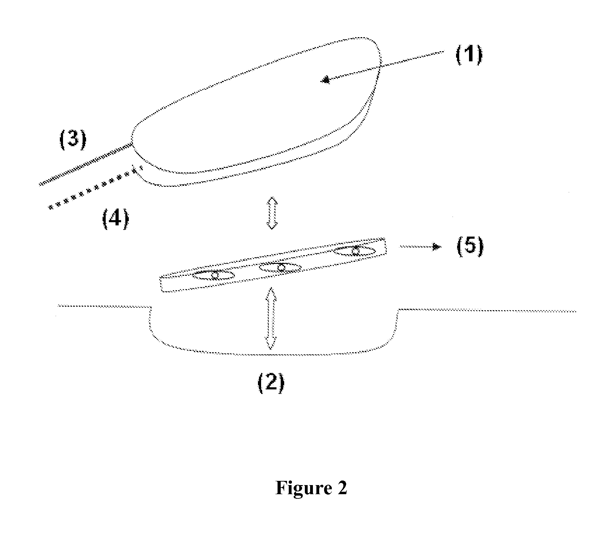

Figure 2 shows a schematic representation of the way in which the flap (1) and the endothelized matrix (5) that will act as a vascularised bridge will be placed in relation to the recipient bed (2). An artery (3) and a vein (4) are again shown in the flap.

Figure 3 is a photograph showing the flap design, on the dorsal side of the ear of a rabbit and with the axis centred in the intermediate caudal vessels. The endothelized matrix is being distributed over the cartilage situated below the flap.

Figure 4 shows immunohistochemical stains of CD32 in treated subjects (part a) and control subjects (b) at 500 magnification.

Figure 5 shows a photograph of vessels of an individual recipient of a flap, treated with the endothelized fibrin gel matrix of the invention, with a positive reaction as regards VEGF in its endothelial cells.

### DETAILED DESCRIPTION OF THE INVENTION

The present invention, therefore, provides a fibrin matrix that has, inside it, endothelial cells transfected with adenoviral vectors and for this reason superproducers of VEGF and/or FGF, which matrix is prepared with the purpose of being used as a connecting vascularised bridge between the recipient bed and a flap.

The endothelial cells have been extracted previously from peripheral veins of an individual of the same species, which can be the actual subject to treat, and are cultured and genetically modified to finally be embedded into a matrix of fibrin gel obtained from plasma, usually also from the actual patient. The genetic modification of the endothelial cells is produced with adenovirus carrying the VEGF and/or FGF genes, in such a way that, *in vivo,* they behave as bioreactors of the aforementioned factors for a limited time only, while the aforementioned cells transfected with the adenovirus-derived vector survive in the fibrin gel. This endothelized matrix, superproducer of proangiogenic factors, has the purpose of acting as a vascularised bridge to induce the development of a functional vascular plexus between the flap and the recipient bed, with the aim of accelerating the adaptation between both. The fibrin gel matrix provides a suitable environment so that growth factors may generate and accumulate in it.

The advantages of using as a vascularised bridge an endothelized matrix composed of a matrix of fibrin gel in which endothelial cells that are superproducers of proangiogenic cells due to having been transfected *in vitro* with adenoviral vectors that contain genes encoding the aforementioned proangiogenic factors are embedded, are as follows:
- The fibrin gel matrix allows the growth and migration of endothelial cells within it. Inside this matrix, genetically modified endothelial cells are capable, *in vitro,* of secreting proangiogenic growth factors (VEGF and FGF) and arranging themselves to form micro-capillaries before being transplanted.
- After transplanting this vascularised bridge, placed between the recipient bed and the flap, the growth factors produced by the genetically modified cells are also capable of inducing the proliferation and migration of new vessels in the recipient bed as well as in the flap itself. The endothelial cells of the vascularised matrix should act as a bridge between both, many of them ending up by being included as part of the vascular stroma which will bind the transplanted tissue to the recipient bed, such that, overall, increases the possibilities of success in the reconnection of the flap.
- The increase in angiogenesis in the transplanted tissues helps to accelerate the incorporation of a flap of skin, muscle, or bone tissue or a combination of these tissues, to the recipient bed.
- The use of this endothelized matrix as a vascularised bridge is of great use not only for reducing thrombotic events that could threaten the survival of the flaps, but also for the treatment of flaps that may have to be used to reconstruct areas treated with radiotherapy, flaps in diabetic patients or smokers (who usually have micro- and/or macrovascular problems), and even to prepare prefabricated flaps.
- The fact that the genetic modification of the endothelized cells is produced by an *in vitro* transfection, before the matrix transplant, ensures that the incorporation of the genetic material is produced specifically in the desired cells and means a clear advantage over the *in vivo* injection of viral vectors, as it decreases the risk of systemic dispersal, allows a continued release effect of growth factors for days and does not require repeat doses.
- The use of adenoviruses as carrier vectors is also an advantage compared to the use of retroviruses, since it avoids the risk of a possible packing of genetic material with oncogenic potential together with inactivated vectors and their possible integration into the host cell in a stable form and blocking other genes.
- The possibility that all the components of the endothelized matrix may be of autologous origin allows that, in those cases, the insertion of the matrix as an endothelized bridge and the corresponding flap may be performed without the need for immunosuppression of the treated subjects.
- On the other hand, the flexibility within the method for obtaining the endothelized matrix allows that the serum used to form the fibrin gel as well the endothelized cells that are transfected and embedded into the matrix could come from a different individual from that who is going to have the flap inserted. Even the species from which the fibrinogen of the fibrin matrix derives can be different. This opens the possibility that matrices may be prepared in advance when they are required urgently, although in these cases it would be advisable to provoke immunosuppression in the subjects in whom the endothelized matrix is implanted as a vascularised bridge.

To achieve this, endothelial cells have to be produced, transfected *in vitro* with an adenoviral vector that enables the expression of VEGF and/or FGF. The aforementioned endothelial cells are extracted from the peripheral nervous system, preferably from the saphenous vein, of the donor. If the endothelized matrix needs to be completely autologous, the donor will have to be the same individual who needs the flap. The extracted vessels are sent in a transport flask with DMEM and an antiseptic solution for their culture and preparation, using, for example, the method described by Del Rio et al, Br J Pharmacol, 120:1360-1366, 1997. Following this method, the endothelial cells are cultured in a suitable medium, such as, the Dulbecco's modified medium: Ham's F12 (1:1) which contains 10% FCS supplemented with glutamax 1, 100 IU/ml penicillin G, 100 µg/ml streptomycin and 0.25 µg/ml amphotericin B.

The *in vitro* genetic transfer is carried out with confluent cultures by incubating them with adenoviral vectors that carry the genes encoding the growth factors (VEGF, FGF) in a serum-poor medium. After two washes with PBS, the cells are incubated in a suitable culture medium, such as, Dulbecco: Ham's F12 (1:1), for a suitable time that, in the case of humans, would be approximately 24 hours. Then, to obtain the individual cells that will form part of the endothelized matrix, they are treated with trypsin/EDTA.

To prepare the endothelized matrix, the method described previously in the international patent application WO 02/072800 can be used. It starts with serum, having platelets and fibroblasts, which is gelled by adding Ca²⁺. Unlike the aforementioned document, in the present invention fibroblasts are not resuspended in the fibrin matrix, nor are the keratinocytes seeded in it, but the type of cells that are resuspended in the matrix are endothelial cells which are obtained as described in the previous paragraph, which are modified genetically. In this way, the matrix of the fibrin gel of the present invention not only acts as a cellular support, but also serves as a vehicle of therapeutic factors produced by the cells.

The fibrin matrix can also be obtained from its blood precursor, fibrinogen, from plasma cryoprecipitates. The cryoprecipitates are obtained in accordance with the standards of the American Association of Blood Banks (Walker RH (ed) Technical Manual, American Association of Blood Banks, Bethesda, MD; 1993; pp. 728-730).

The individual from whom the plasma is extracted may or may not be that in whom the flap is going to be inserted and, together with this flap, the endothelized artificial matrix which is a superproducer of proangiogenic factors. It is preferred that the individual is the same person if it is desired to avoid the need of subjecting the flap recipient to an immunosuppressive treatment. In cases where this is not a fundamental factor, the plasma may come not only from a different individual, but also from a different species. Thus, the fibrinogen source can be, for example, porcine plasma cryoprecipitates.

In any of the cases, to produce the fibrin gel, DMEM which contains 1% FCS and the already transfected endothelial cells are added to the fibrinogen solution. Subsequently, gelification is induced by the addition of CaCl₂ and thrombin. Finally, the mixture is poured over a culture plate or other suitable receptacle and is left to solidify at a suitable temperature, which will be 37°C in the case of fibrin gels originating from humans. The formed gel is covered with a suitable culture medium, (for example, Dulbecco:Ham's F 12 (1:1) and 24-48 hours afterwards it is transplanted over the recipient bed of the flap, in order to act as a vascularised bridge between both. Once the flap is positioned over the endothelized matrix, the edges of the same are sutured to the bed itself, in such a way that the endothelized matrix results homogeneously distributed between both.

As described in more detail below in the corresponding example, surgical experiments performed on animals, specifically rabbits, confirmed the validity of the method and its usefulness in increasing the survival of inserted flaps. Besides, uponr performing a statistical analysis, the results showed that the capillary density and the VEGF expression were significantly better in the treated subjects.

### Example

### Preparation of the endothelial cells

The endothelial cells used to be lodged in the fibrin matrix were endothelial cells from the aorta of New Zealand albino rabbits. These same cells were cultivated after extracting them from the aortic artery of these rabbits under sterile conditions and in a culture medium (5% DMEM, with an antibiotic and an anti-fungicide). The cells were cultured in a Dulbecco's modified medium : Ham's F 12 (1:1), which contains 10% FCS, supplemented with glutamax I, 100 IU/ml penicillin G, 100 µg/ml streptomycin and 0.25 µg/ml amphotericin B.

In the study, cells were used that had been subject to three passages at the most during their culture. The *in vitro* genetic transfer was performed in confluent cultures, by incubation for 3 hours at 37°C with a Group C adenoviral vector which included a gene of VEGF A165, capable of being expressed in endothelial cells in a serum-poor medium. After two washes with PBS, the cells were incubated in a growth medium for 24 hours and subsequently treated with trypsin/EDTA, to obtain individual cells.

### Preparation of the endothelized fibrin matrices

The fibrin gels containing the transfected cells were prepared following the protocol for fibroblast-fibrin gels described in the international patent application WO 02/072800, with modifications. Firstly, fibrinogen from porcine plasma cryoprecipitates was used as a resource for obtaining the fibrin. The cryoprecipitates were obtained in accordance with the standards of the American Association of Blood Banks. To produce the fibrin gel, 3 ml of the fibrinogen solution were added to 12 ml of DMEM in 10% FCS, with 5 x 10⁵ transfected endothelial cells. Then 1 ml of CaCl₂ (0.025 mM, Sigma) was added along with 11 IU bovine thrombin (Sigma). Finally, the mixture was poured into a 75 cm² culture recipient and left to solidify at 37°C. The gel was covered with a culture medium to be used after 24 hours, being kept in a refrigerator at 4°C during this time interval.

### Surgical procedure

The animals in which the flaps were inserted were also New Zealand albino rabbits, although those individual from whom the endothelial cells had been obtained were not used. For this reason, as well as the use of porcine plasma as a fibrin source, immunosuppression had to be provoked in the treated subjects, what in this case was carried out with Sandimmune® (Novartis Pharmaceuticals), using an intraperitoneal dose of 25 mg/kg the day before the operation. This dose was repeated daily in all the subjects of the study, until they were sacrificed.

As regards obtaining the flaps, axial flaps were designed from the dorsal region of the ear of each rabbit, which is based in the intermediate branch of the artery and caudal auricular vein. The proximal edge of each flap was located 4 cm distal to the junction of the median caudal auricular vein with the corresponding caudal vein of the ear. Under aseptic and antiseptic conditions, the edges were infiltrated with local anaesthetic and an incision was made on the edges with a scalpel, trying not to section the vascular pedicle. On the distal edge of the flap, electro-coagulation of the central vessels was performed after isolating them. Using blunt-end scissors, the flap was separated from the cartilaginous tissue, observing the central vessel insertions to the perichondrium. Then, with the aid of a scalpel, the whole flap was removed in a proximal direction. Thanks to a scalpel incision, the vessels in the proximal area of the axial flap were accessed, up to the junction of the median caudal auricular vein with the corresponding caudal vein. At this time the caudal vein was coagulated, to avoid any interference of this vessel with the axial vessel of the flap. The perichondrium was then removed from the cartilage in the exposed area. With the aid of magnifying glasses, lenses and microsurgery tools, the blood vessels were isolated and the nerve fillets that are attached to the central vessels were sectioned, because these contain small accompanying vessels that could nurture the actual flap by themselves.

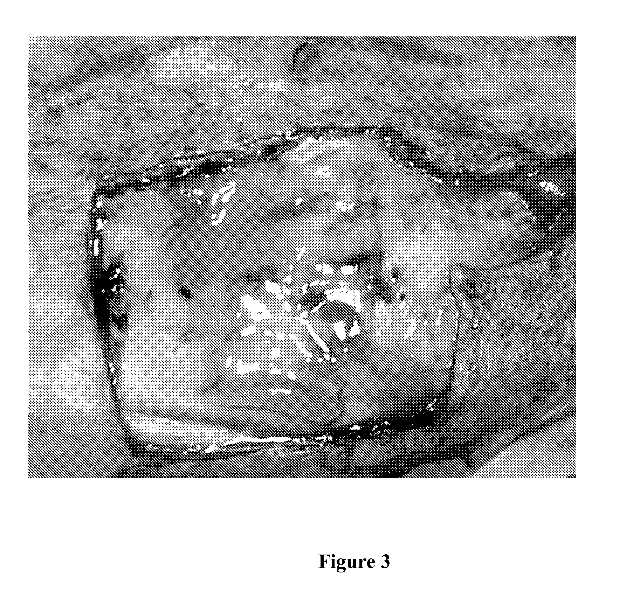

The endothelized fibrin matrix was handled in a laminar flow hood, to remove it from the glass recipient containing it, with the aid of a spatula. The capsule was then covered with sterile paper and was transported to the operating theatre for its implantation over the cartilage with its perichondrium removed. It was ensured that the distribution of the matrix was as homogeneous as possible. The flap was positioned over it. Using a silk suture (4/0), the edges of the same and the incision made to expose the vessels were sutured. An example of the final arrangement is shown in Figure 3.

After surgery, the animals were returned to their cages.

The final surgical act consisted in sectioning the vessels of the axial flap. For this, the same anaesthetic procedure was performed again, although in this case, the infiltration with 2% lidocaine was made at ½ cm from the proximal edge of the flap. An incision was made with a scalpel over the surgical scar itself and with the aid of microsurgery clamp the arterial and venous flow was cut off. Then, the vessels were sectioned and ligated. In group I (control) and II (treated with the VEGF-producer matrix), this procedure was carried out at 5 days from the date of removing the flap. In group III (control) and IV (treated with the VEGF-producer matrix), the sectioning was performed 48 hours after carrying out the initial surgery.

### Treatment of the operated animals

Once the animals were returned to their cages after the first surgery, a daily assessment was made of each subject, making a note of details of the colour of the flaps and its consistency to touch.

Four days after performing the sectioning of the vessels, photographs were taken of them and, after 6 days, the animals were killed with an intravenous overdose of sodium pentothal.

At this time the flaps were extracted and placed in recipients with 10% buffered formaldehyde. After 48 hours, three portions from the proximal, medium and distal areas, respectively of each flap were selected, in such a way that the central vessels were in the centre of the histology section, and were embedded in paraffin.

### Macroscopic and microscopic evaluation

The flap surface that was viable was assessed by planimetry; the values obtained being expressed as percentages. The aforementioned planimetry was performed the day before the animal was sacrificed.

Sections of 3-4 µm were made from the paraffin blocks, which were mounted on silanised slides, with a positive surface charge and a capillary gap of 75 µm (ProbeOn^{™} Plus Slides. Catalogue No. 15-188-52. Fischer Biotech^{c} and ChemMate^{™} Capillary Gap Plus Slides. Code S2024: DAKO A/S Biotek Solutions). After paraffin removal and hydration, the slices were washed in Tris saline (TBS) (0.05 M Tris-HCl; 0.5 M NaCl; pH 7.36). Endogenous peroxidase activity was blocked using 3 % hydrogen peroxide in methyl alcohol for 30 minutes.

With the intention of exposing the highest possible number of epitopes, by unfolding the proteins by denaturation, the sections were subjected to a pre-treatment with heat in a microwave oven at 750 watts, submerged in a citrate buffer (0.01 M citric acid, pH 7), for four periods of five minutes, then leaving them to cool to room temperature.

The non-specific background staining block was done by incubiating non-immune normal goat serum (Code NGS-1, University of Navarre, Pamplona), diluted 1:20, for 30 minutes at room temperature.

The slices were incubated with anti-CD31 antibodies (anti-CD31 mouse monoclonal antibody. Code M 0823. DAKO), with a 1/100 dilution, and anti-VEGF (VEGF (C-1): SC-7269. Santa Cruz Biotechnology, Inc.). The CD31 protein is specific for endothelial cell membranes; therefore, the stainings that can detect those sites to which the anti-CD-31 antibodies will bind enable the blood vessel walls to be visualised and, therefore, show their presence.

After washing in TBS (0.05 M Tris-HCl; 0.5 M NaCl; pH 7.36), it was incubated for 30 minutes at room temperature, with the EnVision^{™} product, peroxidase (DAKO EnVision^{™}. Code No. K4003 anti-rabbit; Code No. K4001 anti-mouse) pre-diluted.

After the final wash in TBS (0.05 M Tris-HCl; 0.5 M NaCl; pH 7.36), the product of the peroxidase reaction was visualised using a commercially prepared 3,3'-diaminobenzidine (DAB) solution in a chromogenic solution, with an imidazole-HCl buffer at pH 7.5 and hydrogen peroxide (DAKO^{c} Liquid DAB+Large Volume Substrate-Chromogen Solution. Code No. K3468), incubating it for five to thirty minutes, at room temperature and pre-diluted.

A pathologist was responsible for carrying out the evaluation of the histological preparations which had been stained with haematoxylin-eosin and the immunohistochemical stains (CD31 and VEGF).

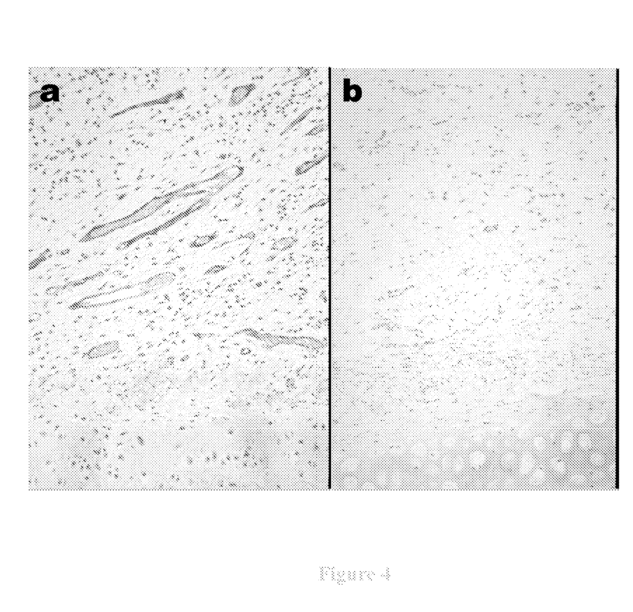

In the CD31 stains, the vessels were counted, at x 500 magnification, in 6 different fields and the mean value of the same is expressed. Those areas where there had been an inflammatory focus were ignored and the areas where there had not been any subcutaneous tissue distortion between the skin appendages themselves and the cartilage were evaluated. Figure 4 shows examples of these CD31 immunohistochemical stains in treated subjects (a) and control subjects (b). The count results are shown in Table 1 subsequently, and corroborate that there is a greater formation of blood vessels in the treated subjects with the matrix of the invention compared to the subjects in the control groups. The associated statistical parameters are shown in Tables 2 and 3.

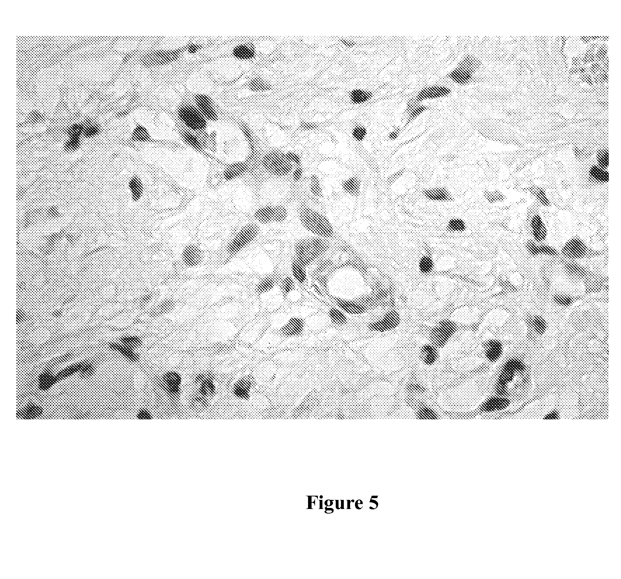

As regards VEGF, those vessels in which the cytoplasm of the endothelial cells was stained were considered positive. The count was made giving a numerical value to the total vessels stained with the antibody in each preparation. An example of vessels stained with a positive reaction for VEGF in endothelial cells in an individual treated with the endothelized fibrin gel matrix of the invention is shown in Figure 5, where the presence of the stained cells demonstrate the presence of VEGF and, therefore, that it was being effectively synthesised. The count results of the stained endothelial cells are shown in Table 1 below. The associated statistical parameters are shown in Tables 2 and 3.

### Results

Table 1 below shows the data of the means and standard deviations corresponding to the survival data, CD31 stains and VEGF stains.

**Table 1. - Values of means and standard deviations corresponding to the survival data, CD31 stains and VEGF stains.**

| | | **MEAN/**STANDARD DEVIATION | | |
|---|---|---|---|---|
| | | SURVIVAL (%) | CD31 Vessels/field* 500 | VEGF Endothelial cells/section |
| *SECTION AT 5 DAYS* | *Group 1 (control)* | **51.25**/45.88 | **5.56**/3.18 | **0.87**/1.12 |
| | *Group II (treatment)* | **95.62/**4.95 | **13.20/**4.54 | **5.62/**3.73 |
| *SECTION AT 48 HOURS* | *Group III (Control)* | **2.50/**7.07 | **2.34/**4.56 | **0.37/**1.06 |
| | *Group IV (treatment)* | **55.62**/38.95 | **5.56**/3.18 | **3.06**/2.95 |

Below, in Tables 2 and 3, the data corresponding to the statistical parameters associated with the previous results are shown, specifically those relative to the Kruskal Wallis (Table 2) and the Whitney-Mann U (Table 3) analysis.

The analysis of variance (ANOVA) is a data analysis technique to examine the significance of the factors (independent variables) in a multifactorial model. The single-factorial model can be obtained from a generalisation of a two-sample test. That is, a test of two samples is that one that starts with the hypothesis that the means of the populations are equal. The ANOVA test will assess the hypothesis that defends that the means of "x" populations are equal.

The Kruskal Wallis test may be used like ANOVA. It is a non-parametric test which is used when the conditions to use the ANOVA test cannot be applied, that is, to contrast the hypothesis that a number of different sized samples originate from the same population. Thus, the Kruskal-Wallis test is a non-parametric method to evaluate the hypothesis that several populations have the same continuous distribution versus the alternative that results tend to be different in one or more populations.

**Table 2. - Kruskal-Wallis parameters**

| **Kruskal Wallis** | **SURVIVAL** | **CD31** | **VEGF** |
|---|---|---|---|
| χ² | 15.50 | 17.11 | 16.38 |
| *P* | 0.001 | 0.001 | 0.001 |

As for Table 3, the Mann-Whitney U test is a non-parametric statistical test that is used when the sample is small or the distribution of the data in the population is free (data not originating from normal populations and with similar variances). This test compares whether two samples of two sub-populations have the same distribution. The observations of both groups are combined and classified according to the average range assigned in case ties are produced. If the position of the populations is identical, the ranges should be randomly mixed in both samples.

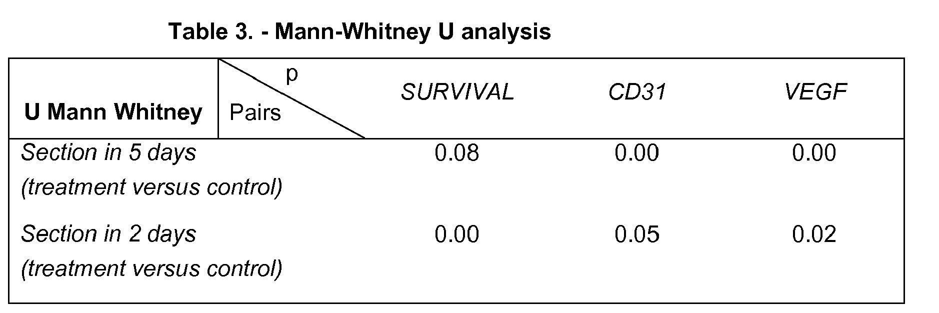

Therefore, according to these data, a survival of the flaps of around 50% was found in treated subjects despite dispensing with the pedicle at 48 hours from being intervened (Group IV), a fact which makes it easier for the flap to necrose, as the blood flow of the flap depends on the pedicle. The survival increased to 95% if the section of the pedicle was performed at 5 days (protocol A). In the non-treated subjects, the survival did not reach 3% after sectioning the pedicle at 48 hours.

The results showing the capillary density and the VEGF expression were also significantly better in the treated subjects.

## Claims

1. An artificial matrix comprising an endothelized fibrin gel which contains endothelial cells embedded in its interior that, in part or in its entirety, has been transfected *in vitro* with one or more adenoviral vectors which comprise in their sequence at least one gene corresponding to a proangiogenic factor capable of overexpression in the mentioned transfected endothelial cells.

2. An artificial matrix comprising an endothelized fibrin gel which is a superproducer of at least one proangiogenic factor according to claim 1, wherein the endothelial cells derive from the venous system of a mammal.

3. An artificial matrix comprising an endothelized fibrin gel which is a superproducer of at least one proangiogenic factor according to claim 2, wherein the endothelial cells specifically derive from the saphenous vein.

4. An artificial matrix comprising an endothelized fibrin gel which is a superproducer of at least one proangiogenic factor according to claim 1, wherein the endothelial cells derive from the arterial system of a mammal.

5. An artificial matrix comprising an endothelized fibrin gel which is a superproducer of at least one proangiogenic factor according to claim 4, wherein the endothelial cells specifically derive from the aortic artery.

6. An artificial matrix comprising an endothelized fibrin gel which is a superproducer of at least one proangiogenic factor according to anyone of claims 1 to 5, wherein the fibrin gel has been formed from fibrinogen present in blood plasma of a mammal.

7. An artificial matrix comprising an endothelized fibrin gel which is a superproducer of at least one proangiogenic factor according to anyone of claims 1 to 5, wherein the fibrin gel has been formed from fibrinogen of plasma cryoprecipitates of a mammal.

8. An artificial matrix comprising an endothelized fibrin gel which is a superproducer of at least one proangiogenic factor according to anyone of claims 1 to 7, wherein at least one adenoviral vector used to transfect the endothelial cells comprises in its nucleotide sequence the coding sequence of the growth factor VEGF capable of being overexpressed in the mentioned endothelial cells.

9. An artificial matrix comprising an endothelized fibrin gel which is a superproducer of at least one proangiogenic factor according to anyone of the claims 1 to 7, wherein at least one adenoviral vector used to transfect the endothelial cells comprises in its nucleotide sequence the coding sequence of the growth factor FGF capable of being overexpressed in the mentioned endothelial cells.

10. A method for obtaining an artificial matrix comprising an endothelized fibrin gel which is a superproducer of at least one proangiogenic factor, **characterised in that** it comprises the steps of:
a) obtaining individualised endothelial cells after having been isolated from a mammal and cultured *in vitro;*
b) transfecting *in vitro* a part or all the mentioned endothelial cells with one or more different adenoviral vectors which comprise in their sequence at least one gene corresponding to a proangiogenic factor capable of being overexpressed in the mentioned endothelial cells;
c) mixing the medium that contains the endothelial cells transfected in the previous step with a solution that contains fibrinogen and stimulating the gelling of the fibrinogen to form fibrin;
d) allowing the mixture from the previous step to stand in a suitable receptacle so that the formation of the fibrin gel matrix is produced in which the endothelial cells transfected with adenoviral vectors have resulted embedded.

11. A method for obtaining an artificial matrix comprising an endothelized fibrin gel which is a superproducer of at least one proangiogenic factor according to claim 10, wherein the stimulation of the gelling of the fibrinogen for the formation of fibrin is provoked by the addition of CaCl₂ and thrombin.

12. A method for obtaining an artificial matrix comprising an endothelized fibrin gel which is a superproducer of at least one proangiogenic factor according to anyone of claims 10 or 11, wherein the endothelial cells derive from the venous system of a mammal.

13. A method for obtaining an artificial matrix comprising an endothelized fibrin gel which is a superproducer of at least one proangiogenic factor according to claim 12, wherein the endothelial cells derive specifically from the saphenous vein.

14. A method for obtaining an artificial matrix comprising an endothelized fibrin gel which is a superproducer of at least one proangiogenic factor according to anyone of claims 10 or 11, wherein the endothelial cells derive from the arterial system of a mammal.

15. A method for obtaining an artificial matrix comprising an endothelized fibrin gel which is a superproducer of at least one proangiogenic factor according to claim 14, wherein the endothelial cells specifically derive from the aortic artery.

16. A method for obtaining an artificial matrix comprising an endothelized fibrin gel which is a superproducer of at least one proangiogenic factor according to anyone of claims 10 to 15, wherein the fibrin gel has been formed from fibrinogen present in blood plasma of a mammal.

17. A method for obtaining an artificial matrix comprising an endothelized fibrin gel which is a superproducer of at least one proangiogenic factor according to anyone of claims 10 to 15, wherein the fibrin gel has been formed from fibrinogen of plasma cryoprecipitates of a mammal.

18. A method for obtaining an artificial matrix comprising an endothelized fibrin gel which is a superproducer of at least one proangiogenic factor according to anyone of claims 10 to 17, wherein at least one adenoviral vector used to transfect the endothelial cells comprises in its nucleotide sequence the coding sequence of the growth factor VEGF capable of being overexpressed in the mentioned endothelial cells.

19. A method for obtaining an artificial matrix comprising an endothelized fibrin gel which is a superproducer of at least one proangiogenic factor according to anyone of claims 10 to 17, wherein at least one adenoviral vector used to transfect the endothelial cells comprises in its nucleotide sequence the coding sequence of the growth factor FGF capable of being overexpressed in the aforementioned endothelial cells.

20. Use of a artificial matrix comprising an endothelized fibrin gel which is a superproducer of at least one proangiogenic factor according to anyone of claims 1 to 9 as a vascularised bridge that is inserted between a flap and a recipient bed of the same in a transplant process between mammals, with the aim of improving the survival of the said flap.

21. Use according to claim 20 wherein the endothelial cells present in the fibrin gel matrix derive from an individual different from the one who receives the flap.

22. Use according to claim 20 wherein the endothelial cells present in the fibrin gel matrix derive from the same individual who receives the flap.

23. Use according to anyone of claims 20 to 22 wherein the individual from which the endothelial cells present in the fibrin gel matrix derive is a non-human mammal.

24. Use according to anyone of claims 20 to 23 wherein the individual who receives the flap is human.

25. Use according to anyone of claims 20 to 23 wherein the individual who receives the flap is a non-human mammal.

26. Use according to anyone of claims 20 to 25 wherein the fibrin gel of the artificial matrix has been formed from fibrinogen present in the blood plasma of an individual different from the one who receives the flap.

27. Use according to anyone of claims 20 to 25 wherein the fibrin gel has been formed from fibrinogen present in the blood plasma of the same individual that receives the flap.

28. Use according to anyone of claims 26 or 27 wherein the individual from whom the blood plasma derives from which the fibrinogen that forms the fibrin gel of the artificial matrix is obtained is a non-human mammal.

29. Use according to anyone of claims 26 to 28 wherein the individual who receives the flap is human.

30. Use according to anyone of claims 26 to 28 wherein the individual who receives the flap is a non-human mammal.

31. Use according to anyone of claims 20 to 25 wherein the fibrin gel of the artificial matrix has been formed from fibrinogen present in blood plasma cryoprecipitates from an individual different from the one who receives the flap.

32. Use according to anyone of claims 20 to 25 wherein the fibrin gel of the artificial matrix has been formed from fibrinogen present in blood plasma cryoprecipitates from the same individual who receives the flap.

33. Use according to anyone of claims 31 or 32 wherein the individual from whom the blood plasma cryoprecipitate derives and from which the fibrinogen that forms the fibrin gel of the artificial matrix is obtained is a non-human mammal.

34. Use according to anyone of claims 31 to 33 wherein the individual who receives the flap is human.

35. Use according to anyone of claims 31 to 33 wherein the individual who receives the flap is a non-human mammal.
